# EUROPEAN PATENT SPECIFICATION

(11) **EP 0 700 283 B1**
(45) Date of publication and mention of the grant of the patent: **25.11.1998**
(21) Application number: 94914189.9
(22) Date of filing: 15.04.1994
(51) Int. Cl.: A61K 9/00

(54) **LIQUID ANTACID COMPOSITIONS**
FLÜSSIGANTAZIDZUSAMMENSETZUNGEN
COMPOSITION LIQUIDE ANTIACIDE

(30) Priority: 26.05.1993 US 67310
(43) Date of publication of application: 13.03.1996
(73) Proprietor: THE PROCTER & GAMBLE COMPANY, Cincinnati, Ohio 45202 (US)
(72) Inventor: CHAPURA, Francis Bernard, Hamilton, OH 45011 (US); FITZGERALD, Jamesina Anne, Hamilton, OH 45013 (US); HUDSON, Jeffrey Scott, Cincinnati, OH 45251 (US); TAYLOR, Charlene Patricia, Cincinnati, OH 45208 (US)
(74) Representative: Brooks, Maxim Courtney
(86) International application number: US9404176
(87) International publication number: WO9427577

(56) References cited:
- FR-A- 2 595 249
- JOURNAL MONDIAL DE PHARMACIE, vol.12, no.4, October 1969 pages 321 - 29 T. J. MCCARTHY 'the influence of insoluble powders on preservatives in solution'
- DRUG DEVELOPMENT AND INDUSTRIAL PHARMACY, vol.13, no.9, 1987, NEW YORK pages 1429 - 46 E. VANHAECKE, ET AL. 'A comparative study of the effectiveness of preservatives in twelve antacid suspensions'

## Description

### BACKGROUND OF THE INVENTION

This invention relates to novel liquid antacid compositions for neutralizing stomach acid in humans and other animals. In particular, it relates to highly efficacious antacid compositions containing calcium carbonate which are aesthetically acceptable and microbially stable.

Pharmaceutical compositions may be produced in a variety of dosage forms, depending upon the desired route of administration of the active material. Liquid suspensions are one preferred dosage form for delivery of antacid active materials which neutralize stomach acid. Liquid antacid suspensions can be generally defined as two-phase systems, wherein a finely divided antacid active, in solid form, is suspended in a liquid medium. These compositions are alkaline, with typical pH values in the range of 7.5 to 9.5.

These compositions must be microbially stable, i.e., resistant to growth of bacteria, molds, and yeast during manufacture. In addition, the compositions must be microbially stable at the time of purchase by the consumer and during the shelf life of the product.

For these reasons, liquid suspension products are typically formulated with preservatives, i.e., compounds having antimicrobial qualities designed to prevent or substantially reduce the growth of microbes in the product. Preservatives known in the art include, for example, alcohols, sorbates, quaternary ammonium compounds, benzoic acid (benzoates) and esters of p-hydroxybenzoic acid (i.e., parabens). However, the preservatives used in liquid antacid products typically impart negative aesthetic qualities to the compositions at the levels used. Such products may have very poor flavor, a chalky and/or numbing mouthfeel, and/or a significant negative after-taste. These aesthetic concerns are particularly important because they may limit consumer acceptability over periods of extended use, thereby limiting consumer compliance with proper treatment regimens.

FR-A-2,595,249 describes aqueous, calcium carbonate suspensions which can be preserved with lower alkyl parahydroxybenzoates such as the methyl or propyl esters.

It has been discovered by the present invention that liquid antacid compositions can be formulated which are microbially stable and aesthetically pleasing. It is therefore an object of the present invention to provide liquid antacid compositions that are both efficacious and aesthetically pleasing to the consumer. It is also an object of the present invention to provide liquid antacid compositions that are microbially stable during the shelf life of the composition.

These and other objects of the present invention will become readily apparent from the detailed description which follows.

All percentages and ratios used herein are by weight, and all measurements are made at 25 C, unless otherwise specified.

### SUMMARY OF THE INVENTION

The present invention relates to liquid antacid compositions. These compositions comprise: from 5% to 40% calcium carbonate; from 0.001% to 0.5% short chain alkyl esters of p-hydroxybenzoic acid selected from methyl, ethyl, propyl and butyl esters and mixtures thereof; from 0% to 0.2% bis-biguanide compound or a pharmaceutically-acceptable salt thereof; from 0.001% to 1% benzyl alcohol; and from 60% to 95% other excipients; and wherein further for those compositions not comprising a bis-biguanide compound, the liquid antacid composition comprises 15% to 40% soluble solids to provide a preservative effect.

The present invention compositions additionally relate to a method for neutralizing excess stomach acid. The method comprises orally administering to a human or lower animal in need of such treatment a safe and effective amount of the liquid antacid composition according to the present invention.

### DETAILED DESCRIPTION OF THE INVENTION

The present invention relates to liquid antacid compositions. These compositions comprise: (a) calcium carbonate; (b) short chain alkyl esters of p-hydroxybenzoic acid selected from methyl, ethyl, propyl and butyl esters and mixtures thereof; (c) benzyl alcohol; (d) optionally, but preferably, bis-biguanide compound or a pharmaceutically-acceptable salt thereof; and (e) other excipients. These compositions are further preferably formulated to contain a 15% to 40% solids content at a level which will increase the shelf stability of the composition.

All components of the present compositions must be pharmaceutically-acceptable. As used herein, a "pharmaceutically-acceptable" component is one that is suitable for use with humans and/or other animals without undue adverse side effects (such as toxicity, irritation and allergic response) commensurate with a reasonable benefit/risk ratio. The components for use in the present compositions, and the preferred amounts to be utilized, are described in detail hereinafter.

### Calcium Carbonate:

The present compositions of the invention contain a safe and effective amount of calcium carbonate as a therapeutically effective antacid active component. Calcium carbonate is effective for neutralizing stomach acid in a human or lower animal. Calcium carbonate typically comprises from 5% to 40%, and preferably from 10% to 30%, by weight of the present compositions.

### Benzyl Alcohol:

The present compositions also comprise benzyl alcohol. Benzyl alcohol is described in more detail in The Merck Index, 10th Edition, published by Merck & Co., No. 1130 (1983). The compositions comprise benzyl alcohol at a level of from 0.001% to 1%, and preferably from 0.01% to 0.5%, by weight of the compositions.

### Short Chain Alkyl Esters of p-Hydroxybenzoit Acid:

The present compositions further comprise short chain alkyl esters of p-hydroxybenzoic acid, which are preservatives also known as "parabens", selected from the methyl, propyl, butyl, and ethyl esters of p-hydroxybenzoic acid. These compositions are approved by the Federal Drug Administration as antimicrobial agents for use in foods and pharmaceuticals. Propylparaben, methylparaben, and mixtures thereof are preferred for use in the present antacid compositions. These materials are described in more detail in The Merck Index, 10th Edition, published (1983) by Merck & Co.: No. 7767 ("propylparaben"), No. 5977 ("methylparaben"), No. 3781 ("ethylparaben"), and No. 1556 ("butylparaben"). These materials are preferably used at levels which reduce or eliminate the negative aesthetics of these parabens in the compositions such that the consumer cannot detect their presence in the present antacid compositions.

The invention compositions typically comprise the short chain alkyl esters of p-hydroxybenzoic acid at a level of from 0.001% to 0.5%; preferably propylparaben at a level of from 0.001% to 0.1%, and methylparaben at a level of from 0.001% to 0.5%, by weight of the compositions. The invention compositions more preferably comprise propylparaben at a level of from 0.001% to 0.05%, and methylparaben at a level of from 0.001% to 0.3%, by weight of the compositions.

### Bis-Biguanide Compounds:

The present antacid compositions optionally, but preferably, may also contain bis-biguanide compounds or the pharmaceutically acceptable salts thereof, at a level of from 0% to 0.2%. Bis-biguanide compounds are described in detail in U.S.-A-3,934,002.

Chlorhexidine and its pharmaceutically acceptable salts are preferred bis-biguanide compounds that are useful in the present compositions. The term "pharmaceutically acceptable salts", as used herein, means salts of the bis-biguanide compounds which have the same general properties as the compounds from which they are derived, and which are acceptable from a toxicity viewpoint. Preferred salts include the acetate, the hydrochloride, and the gluconate salts. The most preferred bis-biguanide compound is chlorhexidine gluconate. Chlorhexidine, and its gluconate salt, are commercially known and described in The Merck Index, 10th Edition, published by Merck & Co., No. 2057 (1983). Chlorhexidine may be used in the present antacid compositions at a level of from 0.0001% to 0.005%, and preferably from 0.0001% to 0.002%, by weight of the composition.

### Other Excipients:

The compositions of the present invention may also contain other excipients that modify the physical characteristics and/or therapeutic effects of the present compositions. The other excipients must not, however, adversely affect the antacid therapeutic efficacy of the calcium carbonate used in the compositions. The compositions typically comprise from 60% to 95%, preferably from 70% to 90%, of other excipients by weight of the compositions.

The excipents, in addition, optionally but preferably are present in the invention compositions at a level which provide an elevated soluble solids content in the composition to enhance microbial stability. Excipients useful in providing an elevated soluble solids content include, for example, glycerin, sorbitol, propylene glycol, mannitol, glucose, sucrose, dextrose, and mixtures thereof. The elevated soluble solids preferably comprise from 15% to 40%, more preferably from 20% to 40%, by weight of the composition.

The invention compositions also preferably contain a suspension system comprising one or more compounds which maintain the calcium carbonate in an essentially uniform aqueous suspension at typical conditions of storage and use. Such suspension systems, suspension agents, and methods for their use include those well known in the art. See, for example, M. Pernarowski, "Solutions, Emulsions and Suspensions" Remington's Pharmaceutical Sciences (A. Osol, editor, 15th Edition, 1975). Suspensions agents useful in the present compositions include xanthan gum, guar gum, cellulose gums, cellulose gum derivatives, magnesium aluminum silicate, carboxy vinyl polymers such as carbopol, and mixtures thereof. The suspension agents typically comprise from 0.1% to 1%, and preferably from 0.15% to 0.35%, by weight of the antacid compositions.

The present compositions also may contain a humectant, such as glycerin and sorbitol, which provides a benefit as a mixing aid and helps to modify the water Activity ("Aw") of the compositions. Glycerin is preferred and commercially available in food grade quality. Glycerin typically comprises from 1% to 15%, and preferably from 3% to 10%, by weight of the antacid compositions.

A safe and effective amount of simethicone is also a preferred therapeutically active component in the present antacid compositions. Simethicone is a mixture of dimethyl polysiloxanes and silica gel suitably purified for pharmaceutical use and is therapeutically effective as an antiflatulent. Simethicone is described in more detail in The Merck Index, 10th Edition, published by Merck & Co., No. 8374 (1983). The present antacid compositions typically comprise simethicone at a level of from 0.1% to 2%, and preferably from 0.3% to 1.0%, by weight of the composition.

The present compositions also most preferably comprise one or more sweetening agents. These include materials such as: water-soluble sweetening agents such as monosaccharides, disaccharides, and polysaccharides including, for example, xylose, ribose, glucose, mannose, galactose, fructose, dextrose, sucrose, maltose, partially hydrolyzed starch or corn syrup solids and sugar alcohols such as sorbitol, xylitol, mannitol and mixtures thereof; water-soluble artificial sweeteners such as the soluble saccharin salts, e.g., sodium or calcium saccharin salts, cyclamate salts, acesulfam-K, and the free acid form of saccharin; or dipeptide based sweeteners such as L-aspartyl-L-phenylalanine methyl ester and materials described in U.S. Pat. No. 3,492,131. In general, the amount of sweetener is primarily a matter of taste preference and will vary with the sweetener selected and with the ingredients in the composition being prepared; except that as noted hereinbefore some of these materials may also be present at an elevated level to provide an elevated soluble solids content in the composition to enhance the microbial stability of the composition.

Preferred in the present compositions is a non-nutritive artificial sweetener such as saccharin, and/or a sugar component such as sucrose. Particularly preferred sweetening agents are sucrose and sorbitol. The present compositions comprise from 5% to 40% of one or more sweetening agents, and more preferably from 10% to 35% of one or more sweetening agents by weight of the antacid compositions.

Preferred other excipients useful in the invention compositions also include colorants, flavorants, other pharmaceutical actives (including other antacid agents), and/or coolants. Preferred coolants in the antacid compositions are N-ethyl-p-menthane-3-carboxamide (known commercially as "WS-3"), 3-l-menthoxypropane-1,2-diol (known commercially as "TK-10"), and mixtures thereof. These coolants are described in WO 92-17164, to Upson et al., published October 15, 1992. TK-10 is also described in U.S. Patent 4,459,425 to Amano et al., issued July 10, 1984; and WS-3 is also described in U.S. Patent 4,136,163 to Watson et al., issued January 23, 1979.

The present compositions typically comprise WS-3 at a level of from 0.001% to 0.05%, and TK-10 at a level of from 0.0001% to 0.1% by weight of the composition. Colorants and flavorants preferably comprise from 0.01% to 1% by weight of the composition.

The compositions of this invention may also contain during the manufacturing process antiseptic agents useful in this process as sanitization aides, but which degrade shortly after making the compositions. Typically these agents are used at levels to provide at least 5 ppm of the agent in the composition during the manufacturing process, and preferably at a level of at least 300 ppm. Preferred are hydrogen peroxide, chloramine, and hypochlorite and its salts (e.g., calcium; sodium; potassium). Such materials are described, for example, in Disinfection, Sterilization and Preservation 3d (S. Block ed., 1983).

Finally, the invention compositions also preferably contain a buffering agent. Buffering agents useful herein include, for example, citric, fumaric, malic, adipic, citric, and tartaric acids and mixtures thereof. Buffering agents typically comprise from 0.05% to 0.5% by weight of the present compositions.

### Method of Treatment

The present Invention compositions additionally relate to a method for neutralizing excess stomach acid. The method of treatment herein comprises orally administering to a human or lower animal in need of such treatment a safe and effective amount of a liquid antacid composition according to the present invention.

The term "safe and effective amount", as used herein, means a quantity of the calcium carbonate-containing liquid antacid composition sufficient to yield the desired antacid efficacy without undue adverse side effects (such as toxicity, irritation or allergic response) commensurate with a reasonable benefit/risk ratio when used in the manner of this invention. The specific safe and effective amount will, obviously, vary with such factors as the particular condition that is being treated, the severity of the condition, the duration of the treatment, the physical condition of the patient, the nature of concurrent therapy (if any), and the specific formulation and optional components employed. However, a patient in need of such treatment will typically receive from about 500mg to about 8000mg of calcium carbonate daily.

The following examples further demonstrate and describe embodiments with the scope of the present invention.

### EXAMPLE 1

| Components | Weight % |
|---|---|
| USP Water | 54.656 |
| Methylparaben | 0.05 |
| Propylparaben | 0.01 |
| Xanthan Gum | 0.12 |
| Guar Gum | 0.09 |
| Calcium carbonate | 12.38 |
| Antifoam C^{a)} | 1.27 |
| Sucrose | 15.0 |
| Sorbitol | 11.0 |
| Glycerin | 5.0 |
| Benzyl Alcohol | 0.2 |
| Citric Acid | 0.15 |
| Coolant | 0.00888 |
| Flavor | 0.0645 |
| Colorant | 0.0014 |

| | |
|---|---|
| a) 30% Simethicone sold by Dow Corning. | |

This composition is prepared by first mixing a portion of the glycerin and all of the benzyl alcohol and heating to 65°C, followed by slowly adding and mixing together the methylparaben and propylparaben. The resulting mixture is added with mixing to a preformed mixture of water, xanthan gum, and guar gum until a uniform mixture is obtained. Then the following ingredients are added slowly in the order: remaining glycerin, sorbitol, Antifoam C, calcium carbonate, citric acid, and sucrose. The flavors and coolants are separately combined and then slowly added to the other ingredients.

Ingestion by a person in need of antacid treatment of 1 tablespoon of this composition delivers 1950 mg of calcium carbonate effective for neutralizing the stomach acid of the person in need of the treatment.

### EXAMPLE 2

| Components | Weight % |
|---|---|
| USP Water | 58.61 |
| Methylparaben | 0.15 |
| Propylparaben | 0.04 |
| Xanthan Gum | 0.12 |
| Guar Gum | 0.09 |
| Calcium Carbonate | 22.61 |
| Antifoam C^{a)} | 2.32 |
| Sucrose | 7.5 |
| Sorbitol | 3.0 |
| Saccharin Sodium | 0.035 |
| Glycerin | 5.0 |
| Benzyl Alcohol | 0.2 |
| Citric Acid | 0.25 |
| Flavor | 0.0645 |
| Coolant | 0.00888 |
| Chlorhexidine Gluconate | 0.001 |
| Colorant | 0.0014 |

| | |
|---|---|
| ^{a)} 30% Simethicone sold by Dow Corning. | |

Example 2 is prepared by a method similar to that described in Example 1.

Ingestion by a person in need of antacid treatment of 1 tablespoon of this composition delivers 3900 mg of calcium carbonate effective for neutralizing the stomach acid of the person in need of the treatment.

## Claims

1. A liquid antacid composition comprising:
a) from 5% to 40% calcium carbonate;
b) from 0.001% to 0.5% short chain alkyl esters of p-hydroxybenzoic acid selected from methyl, ethyl, propyl and butyl esters and mixtures thereof;
c) from 0.001% to 1% benzyl alcohol;
d) from 0% to 0.2% bis-biguanide compound or a pharmaceutically-acceptable salt thereof; and
e) from 60% to 95% other excipients;
and wherein further for those compositions not comprising a bis-biguanide compound, the liquid antacid composition comprises 15% to 40% soluble solids to provide a preservative effect.

2. A liquid antacid composition according to Claim 1 comprising:
from 10% to 30% calcium carbonate; and
from 70% to 90% other excipients.

3. A liquid antacid composition according to Claim 1 or Claim 2 wherein the short chain alkyl esters of parahydroxybenzoic acid are selected from methyl and propyl esters and mixtures thereof.

4. The composition according to Claim 3 wherein the short chain alkyl esters of p-hydroxybenzoic acid are selected from 0.001% to 0.2% propyl ester, 0.001% to 0.5% methyl ester, and mixtures thereof.

5. A composition according to any preceding Claim wherein the bis-biguanide compound is chlorhexidine.

6. A liquid antacid composition according to any preceding Claim comprising:
from 0.0001% to 0.005% chlorhexidine or a pharmaceutically-acceptable salt thereof;
and wherein further the soluble solids content of the liquid antacid composition is within the range of from 20% to 40% by weight of the composition to provide a preservative effect.

7. The composition according to any preceding Claim wherein the excipients which are soluble solids comprise glycerin, sorbitol, propylene glycol, mannitol, glucose, sucrose, dextrose, and mixtures thereof.

8. The composition according to any preceding Claim further comprising simethicone, preferably at a level of from 0.1% to 2% by weight of the composition.

9. The composition according to any preceding Claim comprising chlorhexidine gluconate.

## Patentansprüche

1. Flüssige Antazidzusammensetzung, umfassend:
a) 5 % bis 40 % Calciumcarbonat;
b) 0,001 % bis 0,5 % eines kurzkettigen Alkylesters von p-Hydroxybenzoesäure, welcher unter Methyl-, Ethyl-, Propyl- und Butylestern und Gemischen hievon ausgewählt ist;
c) 0,001 % bis 1 % Benzylalkohol;
d) 0 % bis 0,2 % einer Bisbiguanidverbindung oder eines pharmazeutisch annehmbaren Salzes hievon; und
e) 60 % bis 95 % von anderen Exzipientien;
und worin ferner im Fall jener Zusammensetzungen, welche keine Bisbiguanidverbindung enthalten, die flüssige Antazidzusammensetzung 15 % bis 40 % lösliche Feststoffe enthält, um eine konservierende Wirkung zu gewährleisten.

2. Flüssige Antazidzusammensetzung nach Anspruch 1, umfassend:
10 % bis 30 % Calciumcarbonat; und
70 % bis 90 % von anderen Exzipientien.

3. Flüssige Antazidzusammensetzung nach Anspruch 1 oder 2, worin die kurzkettigen Alkylester von p-Hydroxybenzoesäure unter Methyl- und Propylestern und Gemischen hievon ausgewählt sind.

4. Zusammensetzung nach Anspruch 3, worin die kurzkettigen Alkylester von p-Hydroxybenzoesäure unter 0,001 % bis 0,2 % Propylester, 0,001 % bis 0,5 % Methylester und Gemischen hievon ausgewählt sind.

5. Zusammensetzung nach einem der vorstehenden Ansprüche, worin die Bisbiguanidverbindung Chlorhexidin ist.

6. Flüssige Antazidzusammensetzung nach einem der vorstehenden Ansprüche, umfassend:
0,0001 % bis 0,005 % Chlorhexidin oder eines pharmazeutisch annehmbaren Salzes hievon;
und worin ferner der Gehalt an löslichen Feststoffen in der flüssigen Antazidzusammensetzung im Bereich von 20 Gew.-% bis 40 Gew.-% der Zusammensetzung liegt, um eine konservierende Wirkung zu gewährleisten.

7. Zusammensetzung nach einem der vorstehenden Ansprüche, worin die Exzipientien, welche lösliche Feststoffe sind, Glycerin, Sorbit, Propylenglycol, Mannit, Glucose, Saccharose, Dextrose und Gemische hievon umfassen.

8. Zusammensetzung nach einem der vorstehenden Ansprüche, welche ferner Simethicon, vorzugsweise in einer Menge von 0,1 Gew.-% bis 2 Gew.-% der Zusammensetzung enthält.

9. Zusammensetzung nach einem der vorstehenden Ansprüche, welche Chlorhexidingluconat enthält.

## Revendications

1. Composition liquide antiacide, comprenant:
a) de 5% à 40% de carbonate de calcium;
b) de 0,001% à 0,5% d'esters alkyliques à chaîne courte d'acide p-hydroxybenzoïque choisis parmi les esters méthylique, éthylique, propylique et butylique, et leurs mélanges;
c) de 0,001% à 1% d'alcool benzylique;
d) de 0% à 0,2% de composé bis-biguanide ou d'un de ses sels pharmaceutiquement acceptables; et
e) de 60% à 95% d'autres excipients;
et dans laquelle, en outre, pour les compositions ne comprenant pas de composé bis-biguanide, la composition liquide antiacide comprend 15% à 40% de matières solides solubles pour procurer un effet conservateur.

2. Composition liquide antiacide selon la revendication 1, comprenant:
de 10% à 30% de carbonate de calcium; et
de 70% à 90% d'autres excipients.

3. Composition liquide antiacide selon la revendication 1 ou la revendication 2, dans laquelle les esters alkyliques à chaîne courte d'acide para-hydroxybenzoïque sont choisis parmi les esters méthylique et propylique, et leurs mélanges.

4. Composition selon la revendication 3, dans laquelle les esters alkyliques à chaîne courte d'acide p-hydroxybenzoïque sont choisis parmi 0,001% à 0,2% d'ester propylique, 0,001% à 0,5% d'ester méthylique, et leurs mélanges.

5. Composition selon l'une quelconque des revendications précédentes, dans laquelle le composé bis-biguanide est la chlorhexidine.

6. Composition liquide antiacide selon l'une quelconque des revendications précédentes, comprenant de 0,0001% à 0,005% de chlorhexidine ou d'un de ses sels pharmaceutiquement acceptables;
et dans laquelle, en outre, la teneur en matières solides solubles de la composition liquide antiacide est dans la gamme de 20% à 40% en poids de la composition, pour procurer un effet conservateur.

7. Composition selon l'une quelconque des revendications précédentes, dans laquelle les excipients qui sont des matières solides solubles comprennent la glycérine, le sorbitol, le propylèneglycol, le mannitol, le glucose, le saccharose, le dextrose, et leurs mélanges.

8. Composition selon l'une quelconque des revendications précédentes, comprenant, en outre, de la siméthicone, de préférence à raison de 0,1% à 2% en poids de la composition.

9. Composition selon l'une quelconque des revendications précédentes, comprenant du gluconate de chlorhexidine.
